# EUROPÄISCHE PATENTSCHRIFT

(11) **EP 3 256 078 B1**
(45) Veröffentlichungstag und Bekanntmachung des Hinweises auf die Patenterteilung: **01.04.2020**
(21) Anmeldenummer: 16704009.6
(22) Anmeldetag: 12.02.2016
(51) Int. Cl.: A61F 2/38, A61F 2/36, A61F 2/30, A61F 2/46

(54) **GELENKINTERIMSPROTHESE**
INTERIM JOINT PROSTHESIS
PROTHÈSE ARTICULAIRE PROVISOIRE

(30) Priorität: 13.02.2015 DE 102015202715
(43) Veröffentlichungstag der Anmeldung: 20.12.2017
(73) Patentinhaber: Waldemar Link GmbH & Co. KG, 22339 Hamburg (DE)
(72) Erfinder: DÄNIKE, Andreas, 24558 Henstedt-Ulzburg (DE); JENDRO, Günther, 24568 Kaltenkirchen (DE)
(74) Vertreter: Hoffmann Eitle
(86) Internationale Anmeldenummer: PCT/EP2016/053010
(87) Internationale Veröffentlichungsnummer: WO 2016/128551

(56) Entgegenhaltungen:
- US-A1- 2010 217 401
- US-A1- 2014 194 811
- US-A1- 2014 277 532
- US-A1- 2014 309 745

## Beschreibung

### GEBIET DER ERFINDUNG

Die vorliegende Erfindung betrifft eine in einen Röhrenknochen einsetzbare modulare Gelenkinterimsprothese.

### STAND DER TECHNIK

Bedingt durch die hohe Zuverlässigkeit von Gelenkendoprothesen, insbesondere im Bereich der Hüfte und des Knies, ist ihr Einsatz mittlerweile ein alltäglicher Eingriff. Dennoch kann es vorkommen, dass sich im Bereich einer implantierten Endoprothese aus verschiedenen Gründen Entzündungen entwickeln. Eine solche sogenannte Sepsis kann dabei eine Revision der zuvor implantierten Endoprothese notwendig machen. Ähnliches gilt auch im Falle einer Gewebewucherung, wie z. B. einem Tumor. Mit anderen Worten wird das im Körper befindliche Gelenkimplantat wieder entnommen, da dessen Integration aufgrund der Sepsis oder des Tumors zumindest gefährdet ist. Ist die Sepsis abgeklungen oder die Rekonvaleszenz nach Tumorentnahme weit genug fortgeschritten, kann ein neues Implantat implantiert werden.

Die Sepsis oder Tumore können je nach Schwere notwendig machen, einen mehr oder weniger großen Bereich des umliegenden Knochengewebes zu sezieren. Folglich fehlt nach einer solchen Explantation ein Teil des Stützapparats, was ohne entsprechenden Ersatz zu einer Verkürzung des umliegenden Weichteilgewebes führt. Eine direkte Neuimplantation, um einen solchen Effekt zu verhindern, ist jedoch nicht möglich. Einerseits muss die Sepsis vollständig abklingen, andererseits ist aufgrund der Sektion von Knochengewebe nur schwer vorhersagbar, was für eine Prothese danach implantiert werden kann. Zudem kann die Sektion zunächst einen Aufbau von Knochengewebe erfordern.

Bei der Behandlung der Sepsis ist der Patient ruhiggestellt, während diese durch den Einsatz von Medikamenten, insbesondere Antibiotika, bekämpft wird. Die Ruhigstellung des Patienten führt dabei nicht nur zu einer Verkürzung des umliegenden Weichteilgewebes, sondern auch zu einer starken Schwächung des Muskelgewebes. In der Regel werden vor einer Implantation einer Endoprothese physiotherapeutische Maßnahmen durchgeführt, um das Muskelgewebe sowie den Bänderapparat zu stärken. Dies ist nach Explantation der Prothese und anschließender Ruhigstellung jedoch nur sehr eingeschränkt möglich und vermindert die Erfolgsaussicht der neuen Endprothese nach erfolgter Revision.

Um der Verkürzung des Weichteilgewebes vorzubeugen, wurde deswegen ein Verfahren entwickelt, bei dem der durch die Explantation entstandene Leerraum teilweise durch Knochenzement aufgefüllt wird. Hierzu wird der im Operationssaal angerührte Knochenzement im noch plastischen Zustand an den auszufüllenden Hohlraum angepasst. Um eine optimale Anpassung eines solchen Knochenzementspacers zu gewährleisten, erfolgt diese wenigsten zum Teil durch Anmodellieren am verbleibenden Knochengewebe des Patienten. Da der Knochenzement im plastischen Zustand allerdings noch nicht ausgehärtet ist und somit in ihm weiterhin eine endotherme Aushärtungsreaktion stattfindet, besteht das Risiko durch zu hohe Temperaturen das umliegende Gewebe zu schädigen. So kann die durch den Knochenzement abgegebene Wärmeenergie zu einer Nekrose des umliegenden Knochengewebes führen.

Allerdings muss der Patient auch bei Verwendung eines Knochenzementspacers weiterhin ruhiggestellt werden, da seine Materialeigenschaften und die mechanische Anbindung an das verbleibende Knochengewebe trotz Anmodellierens unzureichend sind. Es handelt sich bei dem Knochenzement also im Wesentlichen um ein Material, welches den Raum des sezierten Knochens einnimmt, jedoch mechanisch nicht in der Lage ist, eine Mobilisierung des Patienten zu ermöglichen.

Dabei spielt auch das Volumen bzw. die Materialstärke eines solchen Spacers eine Rolle, welche beim Aushärten Schwankungen in den mechanischen Eigenschaften sowie der Abmessungen des Knochenzementspacers verursacht. Im Ergebnis kann ein Knochenzementspacer also weder die Stützfunktion noch die Beweglichkeit des explantierten Gelenkersatzes bereitstellen. Daraus folgt, dass zumindest der Schwächung des Muskelgewebes und des Bänderapparats auch bei Verwendung eines Spacers nicht entgegengewirkt werden kann. Dies wirkt sich insbesondere bei einer längeren Verweildauer des Spacers im Patienten von mehreren Wochen bis Monaten negativ aus.

Die US 2014/0277532 A1 offenbart eine temporäre Knieersatzanordnung zwischen einem Femur und einer Tibia und ist die Grundlage für die zweiteilige Form des Anspruchs 1. Die Knieersatzanordnung schließt eine Tibiaplatte, die an der Tibia befestigt ist, und einen Tibiaschaft ein, der sich durch die Platte in die Markhöhle erstreckt und an der Tibiaplatte anliegt. Weiterhin ist eine Femurplatte an dem Femur angebracht und ein Femurschaft erstreckt sich durch die zweite Platte in den Femurmarkraum und liegt an der Femurplatte an. Ein Verriegelungsabstandshalter verbindet die zwei Schäfte. Der Abstandshalter kann längenverstellbar sein oder zur Längenauswahl in mehreren Größen vorgesehen sein.

Die US 2014/0309745 A1 offenbart einen modularen, einstellbaren, prothetischen Hüft-/Schultergelenkabstandshalter, der zur Implantation im Hüft- oder Schultergelenk geeignet ist. Der Abstandshalter weist einen Rahmen auf, der einen drehbaren Zylinder trägt, von dem sich ein Hals erstreckt, wobei das Kugelelement des Gelenks an seinem Ende befestigt ist. Ein Schaft ist mit der Basis des Rahmens verbunden und wird in den Femur bzw. Humerus implantiert.

### ZUSAMMENFASSSUNG DER ERFINDUNG

Es war somit Aufgabe der Erfindung, eine Lösung zur Verfügung zu stellen, welche die Mobilität eines Patienten, vor allem im Interesse eines günstigen Heilungsverlaufs, fördert. Es war weiter Aufgabe der vorliegenden Erfindung, die OP-Zeit zu verringern, welche vor allem durch das Anfertigen des Spacers verlängert wird. Zudem war es Aufgabe, die Knochenzementspacer durch eine Lösung zu ersetzen, welche keine durch die oben beschriebene Hitzeentwicklung hervor gerufenen, nachteiligen Effekte aufweist.

Diese Aufgabe wird durch die in Anspruch 1 definierte Kombination von Merkmalen gelöst. Die abhängigen Ansprüche beschreiben bevorzugte Ausführungsformen der vorliegenden Erfindung.

Zur Lösung dieser Aufgabe stellt die vorliegende Erfindung eine modulare Gelenkinterimsprothese als Ersatz für eine explantierte Gelenkendoprothese bereit. Die Gelenkinterimsprothese ist in einen Röhrenknochen einsetzbar und weist einen Grundkörper mit einem Grundkörperkopplungsabschnitt sowie einen Schaft mit einem Schaftkopplungsabschnitt auf. Hierbei ist der Schaftkopplungsabschnitt lösbar mit dem Grundkörperkopplungsabschnitt koppelbar.

Der Ausdruck "in einen Röhrenknochen einsetzbar", wie hierin verwendet, verdeutlicht, dass nach der Entnahme der zuvor implantierten Gelenkendoprothese im Falle einer notwendigen Revision, beispielsweise aufgrund einer Sepsis, die Interimsprothese an Stelle der entnommenen, d.h. explantierten Prothese tritt und hierzu teilweise in den verbliebenen Röhrenknochen eingreift. Die Interimsprothese wird also dadurch im Körper des Patienten fixiert, dass sie an Stelle der eigentlichen Endoprothese in den Markraum des Röhrenknochens hineinreicht.

Der modulare Aufbau der Gelenkinterimsprothese bedingt durch seine koppelbaren Komponenten ermöglicht hierbei eine individuelle Anpassung der Prothese an den Patienten, was einen Vorteil gegenüber der herkömmlichen Interimslösungen, insbesondere gegossenen Zementprothesen darstellt. Anders als bei "dauerhaften" Endoprothesen ist die individuelle Anpassung also nicht auf den Prothesenschaft und den Gelenkkopf beschränkt. Verglichen mit mit diesen "dauerhaften" Endoprothesen ist also der Prothesenschaft in einen Grundkörper und einen Schaft aufgeteilt.

Bei einer bevorzugten Ausführungsform der Erfindung ist die modulare Gelenkinterimsprothese aus Kunststoff und/oder Metall ausgestaltet. Hierdurch erhält die Gelenkinterimsprothese eine erhöhte Festigkeit gegenüber herkömmlichen Zementprothesen. Ist die Gelenkinterimsprothese bzw. einzelne Bestandteile hiervon aus Metall gefertigt, so enthält das Metall bevorzugt Eisen oder Titan.

Bei einer Ausführungsform der Erfindung besteht die Interimsprothese beispielsweise aus Stahl. Im Falle von Stahl weisen die Gelenkinterimsprothese bzw. die einzelnen Bestandteile hiervon bevorzugt eine Beschichtung aus Titannitrid (TiN) oder Titanniobnitrid (TiNbN) auf. Eine solche Beschichtung vermeidet die Freisetzung von Nickel und/oder Chrom, die in Stahl vorkommen können, und beugt somit dem Auftreten von Allergien vor.

Bei einer weiteren Ausführungsform der Erfindung enthält die Gelenkinterimsprothese bzw. die einzelnen Bestandteile in ihren Oberflächenbereichen Silber, wodurch eine antimikrobielle Wirkung erreicht wird.

Die Oberflächen der Gelenkinterimsprothese bzw. ihrer einzelnen Bestandteile können antiadhäsiv ausgestaltet sein, um ein Einwachsen von Gewebe- oder Knochenmaterial zu unterdrücken. Im Falle von Titan kann hierzu eine Anodisierung die Bildung von Titanoxid vermeiden. Titanoxid kann in Oberflächenbereichen ein Einwachsen von Gewebe oder Knochenmaterial begünstigen. Sofern die Gelenkinterimsprothese bzw. einzelne Bestandteile hiervon aus eisenhaltigen Metallen wie Stahl gefertigt sind, wird eine Beschichtung mit einer antiadhäsiven silikon-haltigen Schicht, beispielsweise Teflon, bevorzugt. Unabhängig vom Material kann eine antiadhäsiv Oberfläche eines Bestandteils auch durch eine glatte oder polierte Oberfläche erreicht werden. Die mittlere Rauheit Rₐ sollte dabei 0,8 µm nicht überschreiten.

Ferner ist es denkbar, die Gelenkinterimsprothese der vorliegenden Erfindung ganz oder teilweise mit einer Antibiotika-haltigen Beschichtung zu versehen. Hierzu kann ein geeignetes Antibiotikum in eine Matrix eingemischt werden, bevor die Antibiotika-haltige Matrix anschließend auf die Oberfläche der Prothese oder auf Oberflächenbereiche hiervon gebracht wird. Als Matrix kommen beispielsweise zementhaltige Verbindungen mit ausreichender Viskosität zum Einsatz, wie z. B. Knochenzement.

Entsprechende Matrixsysteme können auch zum Ausgleich geringer Volumenunterschiede der Gelenkinterimsprothese gegenüber der explantierten Endoprothese und/oder zum Füllen vorhandener Hohlräume, d.h. zu Modulationszwecken verwendet werden. Letzteres kann bei der Fertigung oder auch vor der OP durchgeführt werden, genauso wie eine Beschichtung der Interimsprothese oder Bestandteilen davon mit diesem Material. So kann dem Entstehen von Hitzeschäden vorgebeugt werden. Durch die bevorzugte Verwendung von Metall für die Interimsprothese werden Hitzeschäden aufgrund ihrer thermischen Leitfähigkeit bei dem Ausgleich von Volumenunterschieden ebenfalls vermieden.

Ein grundsätzlicher Vorteil der modularen Gelenkinterimsprothesen besteht also darin, dass diese Prothesen bereits vor ihrem Einsatz, das heißt vor dem Eingriff an dem Patienten, gefertigt werden. Hierdurch unterscheiden sich die erfindungsgemäßen Gelenkinterimsprothesen von den Systemen des Standes der Technik, die im Laufe einer Operation beispielsweise aus Knochenzement, d. h. einem mehrkomponentigen Polymer, gegossen oder modelliert werden. Ein wesentlicher Nachteil solch herkömmlicher Systeme besteht in der oben erläuterten Hitzeentwicklung, die aufgrund chemischer Reaktionen während der Modulierungsphase einsetzt. Hierdurch können Schäden am Körpergewebe verursacht werden, die mit den Systemen der vorliegenden Erfindung vermieden werden.

Aufgrund der höheren Festigkeit weist die modulare Gelenkinterimsprothese im Vergleich zu herkömmlichen Interimsprothesen ferner eine erhöhte Belastbarkeit auf, wodurch die Mobilität des Patienten in dem Zeitraum ermöglicht wird, während dem die Interimsprothese die eigentliche Endoprothese ersetzt.

Ein Grundkörper der modularen Gelenkinterimsprothese ersetzt hierbei wenigstens einen Teil eines Gelenkes, insbesondere einen Schenkelhalsabschnitt einer Hüfte oder ein Kniegelenk bzw. den femoralen oder tibialen Teil hiervon. Mit anderen Worten stellt der Grundkörper einen Platz- oder Abstandshalter dar, der das Gewebe des Patienten in etwa in seiner ursprünglichen Position hält. Hierbei bildet der Grundkörper bevorzugt die Geometrie des ersetzten Gelenkes bzw. Gelenkabschnittes ab und/oder in etwa die Querschnittsgeometrie des sezierten Knochens in einer zur Längsrichtung des Knochens in etwa senkrechten Ebene, sodass es nicht zu einem Schrumpfen des umliegenden Gewebes einschließlich der äußeren Hautschichten kommt. Hierdurch wird erreicht, dass das verbliebene Körpergewebe nach Ausheilung und Entnahme der Interimsprothese den dauerhaften Ersatz, d.h. die eigentliche Endoprothese, wieder problemlos aufnehmen kann.

In einer bevorzugten Ausführungsform der Erfindung ist der Grundkörperkoppelungsabschnitt komplementär zu dem Schaftkopplungsabschnitt ausgeführt. In weiteren bevorzugten Ausführungsformen der Erfindung ersetzt der Grundkörper zumindest einen Teil der Metaphyse und/oder der Grundkörper stützt sich an der Diaphyse bzw. dem meta- -diaphyseren Bereich eines Röhrenknochen ab.

Bei einer besonders bevorzugten Ausführungsform der vorliegenden Erfindung weist der Grundkörper der Gelenkinterimsprothese mindestens zwei Klemmflügel auf, die relativ zum Grundkörper in einer im Wesentlichen senkrechten Richtung zum Schaft verschiebbar sind, wobei jeder Klemmflügel einen Anschlag aufweist, der sich zumindest auf einer Seite des Grundkörpers in der Längsrichtung des Schafts über den Grundkörper hinaus erstreckt.

Durch die Verschiebbarkeit der Anschläge bzw. Klemmflügel wird eine Fixierung des Grundkörpers am Knochen unabhängig von der Lage des Schafts ermöglicht. Die Klemmflügel beugen hierbei einer Rotation des Grundkörpers um die Schaftlängsachse vor.

Bevorzugt sind die Klemmflügel zum Klemmen diametral in einer Ebene angeordnet, die senkrecht zur Längsrichtung des Schafts liegt. Besonders bevorzugt sind die Klemmflügel unabhängig voneinander verschiebbar, sodass die Anschläge der Klemmflügel in unterschiedlichem Abstand vom Schaft mit der Diaphyse bzw. dem meta-diaphysären Bereich eines Gelenks in Kontakt sein können.

Bei einer weiteren bevorzugten Ausführungsform der vorliegenden Erfindung sind die Klemmflügel über mindestens eine, bevorzugt zwei Teleskopstangen verschiebbar. Durch diese bevorzugte Ausführungsform wird ein einfacher Aufbau der Gelenkinterimsprothese gewährleistet, und der Einsatz von zwei Teleskopstangen je Klemmflügel verhindert eine Rotation des Klemmflügels relativ zum Grundkörper. In einer weiteren, alternativen Ausführungsform der Erfindung kann einer Rotation des Klemmflügels relativ zum Grundkörper dadurch begegnet werden, dass eine Teleskopstange mit einem nicht-rotationssymmetrischen Querschnitt verwendet wird.

Bei einer weiteren, ebenfalls besonders bevorzugten Ausführungsform der vorliegenden Erfindung weist mindestens eine Teleskopstange mindestens eines Klemmflügels eine Ausrutschsicherung auf.

Bevorzugt ist diese Ausrutschsicherung als Aussparung in der Längsrichtung der Teleskopstange ausgeführt, in die ein Klemmflügelfixationselement des Grundkörpers eingreift. Hierdurch wird einem Herausfallen des Klemmflügels vorgebeugt, was insbesondere beim Ausrichten des Grundkörpers nach dem Einsatz der modularen Gelenkinterimsprothese in den Patienten vorteilhaft ist.

Das Eingreifen des Klemmflügelfixationselements in eine als Aussparung ausgeführte Ausrutschsicherung gewährleistet ein Verschieben der Teleskopstange bis zu den äußeren Enden der Aussparung, die wie Anschläge wirken. Die Klemmflügel werden erst beim Feststellen des Klemmflügelfixationselements in ihrer Position fixiert.

Gemäß der Erfindung ist der Grundkörperkoppelungsabschnitt des Grundkörpers von der Gelenkinterimsprothese ein Durchgangsloch, in dem der Schaft verschiebbar angeordnet und bevorzugt über ein Schaftfixationselement fixierbar ist. Hierdurch wird eine genaue Anpassung der Gelenkinterimsprothese über das Fixationselement an einen sezierten Knochen gewährleistet. Eine genaue Anpassung der Gelenkinterimsprothese ergibt sich zumindest nicht nur ausschließlich durch die Auswahl der Schaftlänge, sondern auch durch die Möglichkeit, dass der Schaft in dem Durchgansloch verschoben wird. Der Schaft kann hierbei auf beiden Seiten des Grundkörpers hervorstehen und in Abschnitte anliegender Röhrenknochen implantiert sein, d.h. hineinreichen.

Bei einer weiteren bevorzugten Ausführungsform der vorliegenden Erfindung weist der Schaft einen Eingriffsabschnitt für ein Kopplungswerkzeug zumindest entlang eines Abschnitts seiner Länge auf, der durch mindestens zwei parallele sich gegenüberliegende Flächen ausgebildet ist. Hierdurch wird ein Eingriff eines Kopplungswerkzeugs, bevorzugt eines Schraubenschlüssels, über den gesamten Abschnitt gewährleistet.

Besonders bevorzugt erstreckt sich der Abschnitt über wenigstens die Hälfte oder im Wesentlichen über die gesamte Länge, zumindest aber über 80 % der Schaftlänge. Im Zusammenspiel mit dem Schaftfixationselement oder im Falle einer entsprechenden Querschnittsform des Loches kann ein abgeflachter Abschnitt auch als Rotationssicherung dienen. Dies ist selbstverständlich nicht nur für ein Durchgangsloch, sondern auch für ein Sackloch der Fall.

Bei einer weiteren besonders bevorzugten Ausführungsform der vorliegenden Erfindung weist der Grundkörper der Gelenkinterimsprothese einen Gelenkankopplungsabschnitt auf, an dem ein Gelenkteil mit seinem Gelenkkopplungsabschnitt abnehmbar fixierbar ist.

Der Gelenkankopplungsabschnitt erlaubt die Ankopplung an ein Gelenkteil, woraus sich eine Bewegungsfähigkeit der Gelenkinterimsprothese ergibt. Dies geht mit einem Gewinn an Lebensqualität für den Patienten einher. Besonders bevorzugt ist der Gelenkkopplungsabschnitt hierbei auf der Seite in Längsrichtung des Grundkörpers angeordnet, welcher der Seite gegenüberliegt, aus der der Schaft hervorsteht.

Der Gelenkankopplungsabschnitt und der Gelenkkopplungsabschnitt können kegelstumpfförmig ausgeführt sein. Für eine einfache (De)montage kann der Konuswinkel größer gewählt sein als bei normalen Prothesenverbindungen, sodass eine geringere Selbsthemmung der Konusverbindung erreicht wird.

Bei einer bevorzugten Ausführungsform ist an dem Gelenkteil im Bereich des Gelenkkopplungsabschnitts ein Gelenkfixationselement vorgesehen, beispielsweise in Form einer Schraube, welches das Gelenkteil auf dem Gelenkankopplungsabschnitt fixieren kann.

Im Falle einer Konusverbindung ist das Gelenkfixationselement vorzugsweise derart angeordnet, dass die Konusverbindung durch Fixieren des Gelenkfixationselements, beispielsweise durch Anziehen einer Schraube, zusammengedrückt wird.

Bevorzugt ist der Grundkörper winklig ausgeführt. Für den Einsatz der erfindungsgemäßen modularen Gelenkinterimsprothese als Hüftendoprothese steht der Gelenkankopplungsabschnitt (28) bevorzugt in einem Winkel von 135° zu dem Schaft (60). Der Grundkörper ist in diesem Fall also im Wesentlichen als Schenkelhalsbereich ausgeführt, der durch Schaftelemente modular zu einer Hüftprothese ergänzt wird.

Dient die modulare Gelenkinterimsprothese im Wesentlichen als Platzhalter, beispielsweise für ein künstliches Kniegelenk oder einen femoralen oder tibialen Teil hiervon, dann ist der Gelenkkörper im Wesentlichen zylinderförmig ausgeführt und bildet, wie oben beschrieben, in etwa den Querschnitt des explantierten Gelenks oder in etwa den Querschnitt des Knochens in diesem Bereich ab.

Bei einer weiteren bevorzugten Ausführungsform der Erfindung weist die Gelenkinterimsprothese zwei Grundkörper auf. Die zwei Grundkörper einer solchen bevorzugten Gelenkinterimsprothese können zwei Gelenke, d.h. zwei künstliche Gelenke, die einem Patienten entnommen werden mussten, ersetzen und sind bevorzugt über wenigstens einen Schaft miteinander verbunden.

Eine solche Gelenkinterimsprothese erlaubt den Ersatz also auch in Fällen, bei denen ein herkömmlicher Knochenzementplatzhalter nicht mehr einsetzbar wäre. Beispielsweise können Gelenkinterimsprothesen einer solchen bevorzugten Ausführungsform einen Großteil eines Röhrenknochens, wenn nicht sogar einen gesamten Röhrenknochen, ersetzen. In einer bevorzugten Ausführungsform der Erfindung handelt es sich bei der Gelenkinterimsprothese also um einen zeitweisen Ersatz für ein Hüft- und Kniegelenk.

Bei einer weiteren Ausführungsform der Erfindung weist die Gelenkinterimsprothese ferner einen Schaft mit einer Auflage bzw. einen Auflageschaft auf, wobei der Auflageschaft ein distales und ein proximales Ende aufweist, zwischen denen ein Auflageflansch angeordnet ist, der sich ausgehend vom Auflageschaft radial nach außen erstreckt. Der Auflageschaft hat einen Durchmesser, der größer ist als der mindestens eine Schaftkopplungsabschnitt und ist so bemessen, dass er auf einem sezierten Querschnitt des Röhrenknochens aufliegen kann. Der Auflageschaft ist insbesonder für den Bereich der Diaphyse vorgesehen. Mit anderen Worten ermöglicht der Auflageschaft ein gutes Abstützen der Gelenkinterimsprothese auf einem Röhrenknochen und verhindert ein zu tiefes Eindringen des Schaftes in den Röhrenknochen.

Bei einer weiteren bevorzugten Ausführungsform der Erfindung weisen der Schaft (60) und der Auflageschaft (70) an ihrem proximalen und distalen Ende zueinander komplementäre Gewinde bzw. Gewindeverbinderauf sowie mindestens einen Eingriffsabschnitt für ein Kopplungswerkzeug. Durch diese bevorzugte Ausgestaltung der erfindungsgemäßen Gelenkinterimsprothese wird gewährleistet, dass verschiedene Schäfte und verschiedene Grundkörper miteinander kombinierbar sind. Der Kopplungsabschnitt des Grundkörpers kann hierfür ein Durchgangsloch darstellen oder in Form eines weiblichen oder männlichen Gewindes ausgestaltet sein. Diese Verbindungselemente sind in einer bevorzugten Ausführungsform der Erfindung ein männliches Verbindungselement auf einer Seite des Schaftes und ein weibliches Verbindungselement auf einer anderen Seite des jeweiligen Schafts oder auch Grundkörpers. Unter Gewindeverbindern versteht die vorliegende Erfindung sowohl Verbindungselemente mit einem Gewinde als auch mit einem Bajonettverschluss.

Das vorstehend beschriebene Kopplungswerkzeug zum Anziehen der Gewindeverbinder ist bevorzugt ein Schraubenschlüssel mit zueinander parallelen Eingriffsflächen.

Desweiteren enthält die Vorliegende Beschreibung Verfahren zur Behandlung eines Patienten mit einer Gelenkendoprothese umfassend die Schritte: (i) Entfernen der Gelenkendoprothese aus dem Patienten; und (ii) Einsetzen einer Gelenkinterimsprothese wie hierin beschrieben. Weiterhin umfasst das Verfahren vorzugsweise den Schritt (iii) des Entfernens der Gelenkinterimsprothese.

Die modulare Gelenkinterimsprothese wie hierin beschrieben dient als zwischenzeitlicher Ersatz einer Endoprothese in einem Patienten, bevorzugt einem Menschen. Die Beschreibung betrifft insofern auch Verfahren zur chirurgischen und/oder therapeutischen Behandlung eines menschlichen oder tierischen Körpers, bei dem eine bestehende Gelenkendoprothese zunächst entnommen und anschließend durch die hierin beschriebene Gelenkinterimsprothese für einen bestimmten Zeitraum ersetzt wird. Das Verfahren zur chirurgischen oder therapeutischen Behandlung eignet sich hervorragend bei Patienten mit einer akuten Sepsis im Bereich der Gelenkendoprothese.

### KURZE BESCHREIBUNG DER FIGUREN

Mit Hilfe der folgenden Figuren und Beschreibung werden Ausführungsbeispiele für ein besseres Verständnis zur vorliegenden Erfindung im Detail erörtert. Hierfür werden die in den Figuren ersichtlichen Merkmale mit Bezugszeichen gekennzeichnet. Dabei werden bei unterschiedlichen Ausführungsbeispielen gleiche Bezugszeichen verwendet, sofern sich die Merkmale in diesen Ausführungsbeispielen gleichen oder eine gleiche Wirkung erzielen.
Figur 1 zeigt einen Grundkörper der erfindungsgemäßen Gelenkinterimsprothese für ein Knie (Femur und/oder Tibia), der zwei Klemmflügel mit Anschlägen aufweist.
Figur 2 zeigt den Grundkörper in vergrößerter Ansicht mit eingesetzten Klemmflügeln.
Figur 3 zeigt eine Vorderansicht des Grundkörpers.
Figur 4 zeigt ein Gelenkteil, welches über einen Gelenkkopplungsabschnitt an einem Grundkörper der Gelenkinterimsprothese fixierbar ist.
Figur 5 zeigt eine erfindungsgemäße modulare Gelenkinterimsprothese mit zwei Grundkörpern.
Figur 6 zeigt einen Schaft und einen mit diesem Schaft verbundenen Grundkörper.

Die Figuren 7 und 8 zeigen jeweils ein Set verschiedener Komponenten der modularen Gelenkinterimsprothese wie hierin beschrieben.

### AUSFÜHRLICHE BESCHREIBUNG BEVORZUGTER AUSFÜHRUNGSFORMEN

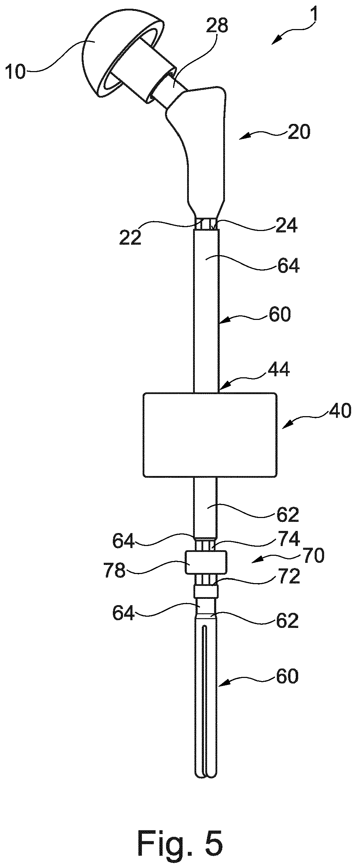

Figur 5 zeigt ein Beispiel für einen Aufbau einer erfindungsgemäßen modular aufgebauten Gelenkinterimsprothese 1, die für den Ersatz einer explantierten Gelenkendoprothese (nicht gezeigt) vorgesehen ist. Die Gelenkinterimsprothese 1 weist zwei Grundkörper 20, 40 auf. Es handelt sich somit bei dem Aufbau dieser Gelenkinterimsprothese 1 um einer Ausführung, die für den Ersatz von zwei explantierten künstlichen Gelenken vorgesehen ist. In dem in Figur 5 gezeigten speziellen Fall handelt es sich dabei um den Ersatz einer Hüftprothese und eines Kniegelenks.

Wie bei der in Figur 5 dargestellten Interimsprothese 1 zu erkennen ist, ermöglicht diese dem Patienten ein gewisses Maß an Mobilität, da das Gelenkteil 10 in der nicht gezeigten Hüftpfanne des Patienten im Acetabulum geführt wird und so seine Funktion als Gelenk erhalten bleibt. Hingegen ist der Grundkörper 40, der als Ersatz für ein explantiertes künstliches Kniegelenk dient, biegesteif und lässt somit keine gelenkige Bewegung zu. Allerdings kann der Patient sein Bein dank des an dem Grundkörper 20 befindlichen Gelenkteils 10 aus der Hüfte heraus bewegen. Folglich ermöglicht die in Figur 5 gezeigte Ausführungsform eines Interimsgelenks 1 zum einen physiotherapeutische Maßnahmen und zum anderen erlaubt dieser Aufbau dem Patienten ein gewisses Maß an Bewegungsfreiheit. Dies wirkt sich positiv auf die Muskulatur und den Bänderapparat aus.

Gleichzeitig sorgt der Grundkörper 20 wie auch der Grundkörper 40 dafür, dass sich das umliegende Weichteilgewebe weder in Längsrichtung des Beins noch in dessen Querrichtung so stark verkürzen kann. Damit wird eine Neuimplantation eines dauerhaften Gelenkersatzes nicht behindert.

Dabei ersetzt der Grundkörper 20 den angewinkelten Bereich des Femurs im Bereich des Schenkelhalses. Für die erfindungsgemäße Interimsprothese wird für diesen Winkel bevorzugt 135° gewählt.

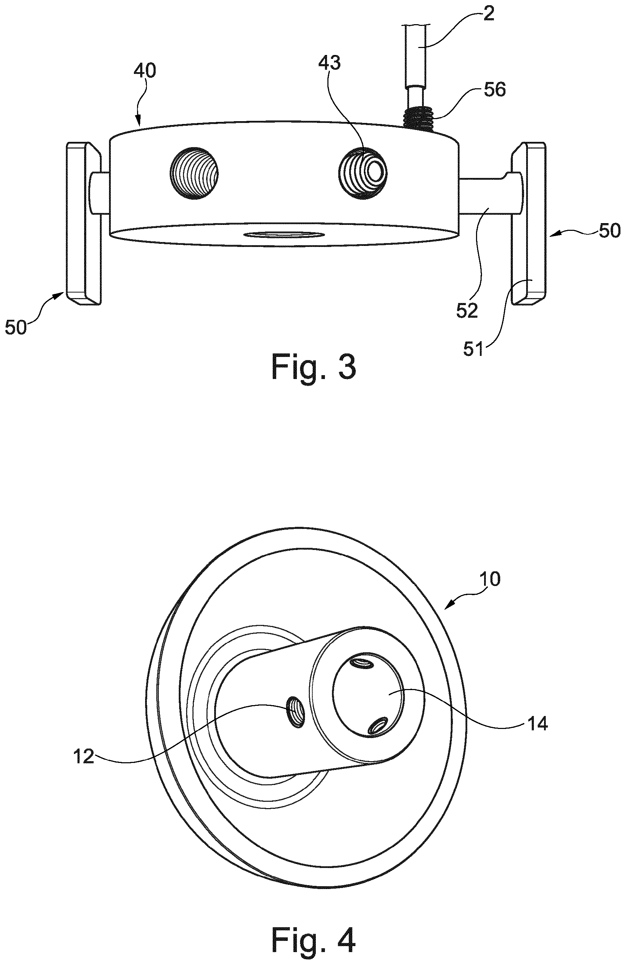

Weist ein Grundkörper 20, 40 ein Gelenkteil 10 auf, so ist für dessen Ankopplung an dem Grundkörper 20, 40 ein Gelenkankopplungsabschnitt 28 vorgesehen. Dieser Gelenkankopplungsabschnitt ist vorzugsweise konusförmig ausgebildet und wurde bereits oben näher erläutert. Folglich weist das Gelenkteil 10 bei einer solchen Ausführungsform eine konusförmige Innenfläche 14 auf (Figur 4).

Weiterhin kann an dem Gelenkteil 10 mindestens ein Gelenkfixationselement 12 vorgesehen sein, um das Gelenkteil 10 an dem Gelenkankopplungsabschnitt 28 des Grundkörpers 20 zu fixieren. Vorteilhafter Weise ist das mindestens eine Gelenkfixationselement 12 so angeordnet, dass dessen Feststellen bzw. Festziehen den konusförmigen Gelenkankopplungsabschnitt 28 in den entsprechenden Gelenkkopplungsabschnitt 14 des Gelenkteils 10 drückt und so eine spielfreie Verbindung sicherstellt. Dies kann beispielsweise durch eine spitz zulaufende Schraube als Gelenkfixationselement 12 und eine entsprechend spitz ausgeführte Aussparung in der Oberfläche des Gelenkankopplungsabschnitts 28 erreicht werden. Ist mehr als ein Gelenkfixationselement 12, so sind diese bevorzugt gleichmäßig um den Umfang des Gelenkkopplungsabschnitts verteilt.

Um den Grundkörper 20, 40 mit einem Schaft 60 zu koppeln, werden insbesondere zwei Ankopplungsmöglichkeiten bevorzugt. Bei der ersten, jedoch nicht im Hauptanspruch enthaltenen, Ankopplungsmöglichkeit weist der Grundkörper 20, 40 in Längsrichtung an zumindest einem Ende einen Grundkörperkopplungsabschnitt 24, 44 auf. Dabei weist der Grundkörperkopplungsabschnitt 24, 44 für das Verbinden bevorzugt ein Gewinde auf. Hierzu zählt im Rahmen der Möglichkeiten auch ein Bajonett. An dem dazu komplementären Schaftkopplungsabschnitt 64 eines mit dem Grundkörper 20, 40 verbindbaren Schafts 60 befindet sich dementsprechend ein komplementäres Gewinde, sodass der Grundkörper 20, 40 und der Schaft 60 miteinander gekoppelt werden können.

Zum Anziehen der Verbindung zwischen einem Schaft 60 und einem Grundkörper 20, 40 ist zudem bevorzugt ein Eingriffsabschnitt 22 an dem Grundkörper 20, 40 und ein Eingriffsabschnitt 62, 72 an dem Schaft 60, 70 vorgesehen. Wie oben beschrieben kann der Eingriffsabschnitt dabei über im Wesentlichen die gesamte Länge des Schafts 60, mindestens aber über 50 %, 60 %, 70 %, 80 % und bevorzugt mindestens 90 % vorgesehen sein.

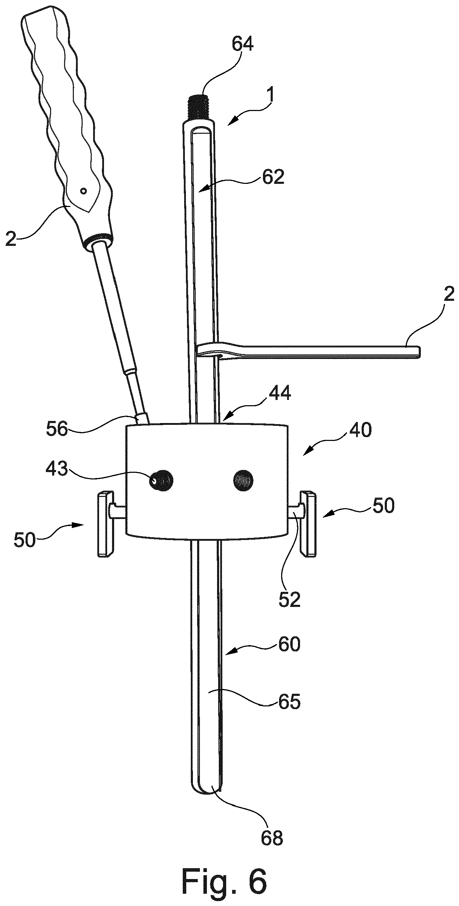

Bei dem Eingriffsabschnitt 22, 62, 72 handelt es sich zudem bevorzugt um zwei zueinander parallele Eingriffsflächen 65 (Figur 6), in die ein Kopplungswerkzeug 2 eingreifen kann. In dem gezeigten Ausführungsbeispiel ist das Kopplungswerkzeug 2 in der Form eines Schraubenschlüssels ausgeführt.

Befinden sich in Längsrichtung an beiden Enden eines Grundkörpers 20, 40 und/oder eines Schafts 60, 70 ein Kopplungsabschnitt, so ist der Kopplungsabschnitt der einen Seite komplementär zu dem Kopplungsabschnitt auf der anderen Seite ausgeführt. Im Falle eines Gewindes befindet sich das Außengewinde dementsprechend auf einer Seite des Grundkörpers 20, 40 bzw. des Schafts 60, 70 und das Innengewinde auf der jeweils anderen Seite desselben Grundkörpers 20, 40 bzw. des Schafts 60. Folglich sind die Schäfte 60, 70 und Grundkörper 20, 40 beliebig untereinander austauschbar.

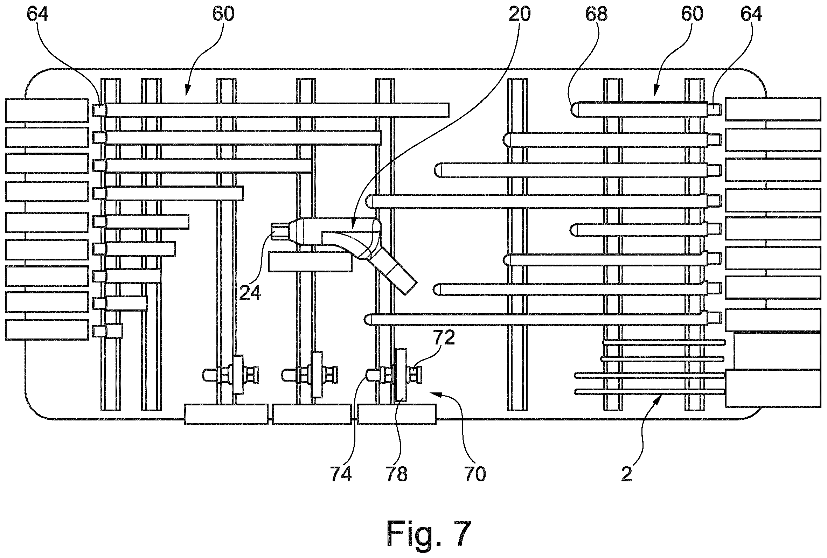

Unter Bezugnahme auf Figur 7 ist dies für den Schaft 60 und den Auflageschaft 70 gezeigt. So ist auf der einen Seite der Schäfte 60, 70 der männliche Kopplungsabschnitt 64 bzw. 74 gezeigt. Der dazu komplementäre weibliche Kopplungsabschnitt 64, 74 auf der entgegengesetzten Seite der Schäfte 60, 70 befindet sich in der Stirnfläche der Schäfte und ist in Figur 7 nicht sichtbar.

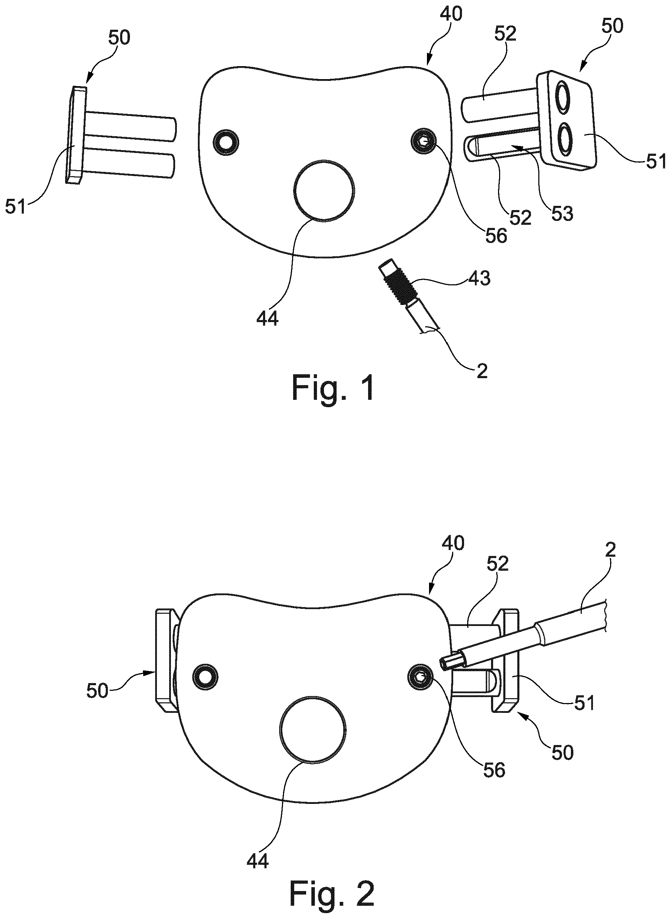

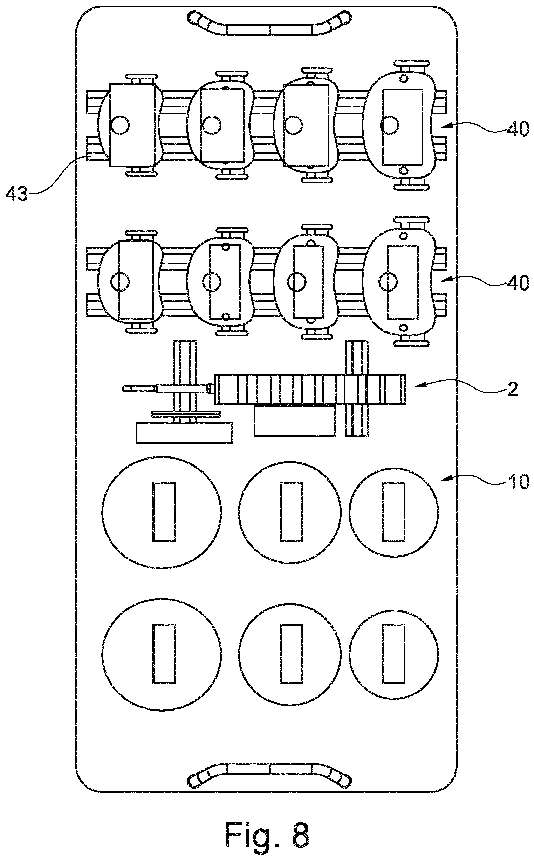

Bei der zweiten, dem Hauptanspruch entsprechenden, Ankopplungsmöglichkeit ist in dem Grundkörper 20, 40 zumindest auf einer Seite ein Loch 44 vorgesehen, in das der Schaft 60, 70 einführbar ist. Wie oben beschrieben, handelt es sich bei dem Loch 44 um ein Durchgangsloch (vgl. auch Figuren 1 bis 3, 6 und 8). Ein Schaft 60, 70 kann dann über mindestens ein Schaftfixationselement 43 fixiert werden.

Da es sich um ein Durchgangsloch 44 handelt, kann der Schaft 60 beidseitig von dem Grundkörper 20, 40 hervorstehen (Figuren 5 und 6). Eine solche Anordnung wird sowohl in Figur 5 als auch in Figur 6 gezeigt. Bei beiden Figuren ist ein Grundkörper 40 als Ersatz für ein künstliches Kniegelenk vorgesehen. In diesen Fällen steht derselbe Schaft 60 in Längsrichtung beidseitig von dem Grundkörper 40 hervor. Dadurch ist es möglich, den in das Durchgangsloch 44 eingeführten Schaft 60 in die beiden anliegenden Röhrenknochen einzuführen und so den Grundkörper 40 lateral zu fixieren. Ist der Grundkörper 40 beispielsweise für den Ersatz eines künstlichen Kniegelenks vorgesehen, so befindet sich eine Seite des Schafts 60 im Femur, während die andere Seite des Schafts 60 in der Tibia angeordnet ist. Ein solcher Fall ist in der Figur 5 gezeigt. Die Verschiebbarkeit eines Schafts 60 in dem Grundkörper 20, 40 aufgrund eines Durchgangslochs 44 hat dabei die oben bereits beschriebenen Vorteile.

In dem in Figur 5 gezeigten Ausführungsbeispiel ist der im Durchgangsloch 44 des Grundkörpers 40 eingeführte Schaft 60 zwar möglicherweise für das Einführen in Abschnitte eines Röhrenknochen vorgesehen, jedoch nicht um die Interimsprothese 1 lateral zu sichern. Stattdessen ist die in Figur 5 gezeigte Interimsprothese 1 für einen Fall vorgesehen, bei dem der gesamte Femur entnommen worden ist, genauso wie der proximalen Teil der Tibia. Dadurch stützt sich keiner der Grundkörper 20, 40 mit ihren in Längsrichtung ausgerichteten Flächen auf einem Röhrenknochen ab.

Um dennoch eine gewisse Verankerung in einem Röhrenknochen zu ermöglichen, ist die Interimsprothese 1 über ihren am distalen Ende angebrachten Schaft 60 in das distale Ende einer nicht gezeigten Tibia einführbar. Um ein Durchrutschen des Schafts 60 zu verhindern, ist ein Auflageschaft 70 vorgesehen, der über die oben beschriebenen komplementären Verbinder 64, 74 zwischen die beiden Schäfte 60 eingefügt ist. Der sich radial nach außen erstreckende Auflageflansch 78 des Auflageschafts 70 liegt dann auf der proximalen Schnittfläche der Tibia auf, übernimmt damit die Rolle der oben genannten Flächen der Grundkörper 20, 40, und verhindert somit ein Durchrutschen. Da die in Figur 5 gezeigte Interimsprothese 1 für den zuvor erwähnten Fall vorgesehen ist, weist der Grundkörper 40 keine bereits oben und nachfolgend näher beschriebenen Klemmflügel 50 auf.

Steht ein Schaft 60 hingegen nur auf einer Seite eines Grundkörpers 20, 40 hervor, so dient die Interimsprothese nur für den Ersatz einer Gelenkseite eines zuvor explantierten künstlichen Gelenks. So würde in der Figur 5 der Grundkörper 20 bei Ankopplung des Schafts 60 über ein Loch nur den femoralen Teil des Hüftgelenks ersetzen. Gleiches gilt für den Ersatz des tibialen bzw. femoralen Teils eines Kniegelenks. Bei einem einseitigen Ersatz weist der Grundkörper 40 eine entsprechend kürzere Länge auf.

Im Allgemeinen bewegt sich für den Grundkörper 40, der in den gezeigten Ausführungsformen für den Ersatz eines Kniegelenks (Figuren 5 und 6) oder des tibialen Teils eines Kniegelenks (vergleiche Figuren 1 bis 3) vorgesehen ist, dessen Länge in einem Bereich von 10 bis 80 mm. Die Abmessung des Grundkörpers 40 bei den gezeigten Ausführungsformen bewegt sich in lateraler Richtung zwischen 40 und 90 mm und in der senkrecht dazu liegenden Richtung zwischen 35 bis 55 mm.

Der Schaft weist bevorzugt einen über seine Länge gleich bleibenden Querschnitt auf. Der Begriff "im Wesentlichen" schließt dabei möglicherweise vorhandene Eingriffsflächen 62, wie in Figur 6 gezeigt, ein. Der Querschnitt des Schafts 60 ist jedoch so ausgeführt, dass er durch den diaphysären Bereich eines Röhrenknochens ein- bzw. durchführbar ist. Hierfür weist der Schaft 60 einen Durchmesser von 8 bis 15 mm, bevorzugt 10 bis 12 mm auf.

Der Grundkörper 20, 40 in Übereinstimmung mit der vorliegenden Erfindung zeichnet sich dadurch aus, dass er im Gegensatz dazu bevorzugt nicht in den diaphysären Bereich des anliegenden Röhrenknochen einführbar ist. In dem Querschnitt senkrecht zu seiner Längsrichtung folgt die Außenkontur des bevorzugt zylindrisch ausgeführten Grundkörpers 40 in seiner bevorzugten Ausführungsform als Ersatz für zumindest einen Teil eines Kniegelenks im Wesentlichen der äußeren Form im metaphysären Bereich einer Tibia im gleichen Gelenkabschnitt (Figuren 1 und 2).

Ein Grundkörper 20, 40 kann zudem Klemmflügel 50 aufweisen (Figuren 1 bis 3 und 6). Die Klemmflügel 50 sind bezogen auf den Querschnitts des Grundkörpers 40 senkrecht zu dessen Längsrichtung vorzugsweise (diametral) gegenüberliegend angeordnet.

Sie werden in einer Ausführungsform mittels einer Teleskopstange 52 geführt, die in einer Öffnung bzw. einem Loch des Grundkörpers 40 verschiebbar angeordnet ist. Somit kann ein Klemmflügel 50 ähnlich wie eine Spannbacke bei einem Schraubstock aus dem Grundkörper 40 herausgezogen und hineingeschoben werden.

Wie in Figur 3 zu erkennen weist der Klemmflügel 50 einen Anschlagbereich 51 auf, der im eingesetzten Zustand der Interimsprothese 1 mit seiner dem Grundkörper 40 zugewandten Fläche an den anliegenden Röhrenknochen anschlägt. Da dies für beide sich gegenüberliegenden Klemmflügel 50 gilt, wird der dazwischen liegende Bereich, das heißt bevorzugt der metaphysäre Bereich, eingeklemmt. Der dabei durch den in Längsrichtung des Grundkörpers 40 über den Grundkörper 40 hervorstehende Teil des Anschlagbereichs 51 hervorgerufene Formschluss mit dem Umfang des anliegenden Röhrenknochen sichert den Grundkörper 40 gegen eine Rotation um seine Längsachse.

Die Klemmflügel 50 werden nach ihrer Justierung durch das Klemmflügelfixationselement 56 in dem Grundkörper 40 fixiert. Zudem kann bei den Klemmflügeln 50 eine oben bereits näher beschriebene Ausrutschsicherung 53 vorgesehen sein, die in der in den Figuren 1 bis 3 gezeigten Ausführungsformen als in der Längsrichtung einer Teleskopstange ausgeführte Aussparung ausgebildet ist.

Das äußerste Ende eines Schafts 60 kann als abgerundeter Endabschnitt 68 ausgeführt sein (Figur 5 bis 7) .

In allen erfindungsgemäßen Ausführungsformen ermöglicht die Interimsprothese aufgrund ihres modularen Aufbaus eine patientenspezifische Lösung. Durch ihre mechanische Belastbarkeit und je nach Ausführung auch Gelenkigkeit wird einem Patienten zudem eine eingeschränkte Mobilität ermöglicht. Dies wirkt sich positiv auf die Lebensqualität des Patienten während der Krankheitsphase aus und verbessert zudem die Vorbereitung und damit die Erfolgsaussichten einer Revision.

### BEZUGSZEICHEN

- 1: Interimsprothese
- 2: Kopplungswerkzeug
- 10: Gelenkteil
- 12: Gelenkfixationselement
- 14: Gelenkkopplungsabschnitt
- 20: Grundkörper
- 22: Eingriffsabschnitt für Kopplungswerkzeug
- 24: Grundkörperkopplungsabschnitt für einen Schaft
- 28: Gelenkankopplungsabschnitt
- 40: Grundkörper für Knie
- 43: Schaftfixationselement
- 44: Grundkörperkopplungsabschnitt
- 50: Klemmflügel
- 51: Anschlag
- 52: Teleskopstange
- 53: Ausrutschsicherung
- 56: Klemmflügelfixationselement
- 60: Schaft
- 62: Eingriffsabschnitt für Kopplungswerkzeug
- 64: Schaftkopplungsabschnitt
- 65: Eingriffsfläche eines Eingriffsabschnitts 62
- 68: abgerundeter Endabschnitt
- 70: Auflageschaft
- 72: Eingriffsabschnitt
- 74: Kopplungsabschnitt des Auflageschafts
- 78: Auflageflansch

## Patentansprüche

1. In einen Röhrenknochen einsetzbare Gelenkinterimsprothese (1) für den Ersatz einer zu explantierenden Gelenkendoprothese, wobei die Gelenkinterimsprothese einen Grundkörper (40) mit einem Grundkörperkopplungsabschnitt (44) und einen Schaft (60) mit einem Schaftkopplungsabschnitt (64) aufweist und der Schaftkopplungsabschnitt (64) lösbar mit dem Grundkörperkopplungsabschnitt (44) koppelbar ist, **dadurch gekennzeichnet, dass** der Grundkörperkopplungsabschnitt (44) zum Ankoppeln ein Durchgangsloch aufweist, in dem der Schaft verschiebbar angeordnet ist, sodass der Schaft auf beiden Seiten des Grundkörpers (40) hervorstehen kann.

2. Gelenkinterimsprothese (1) nach Anspruch 1, bei welcher der Grundkörper (40) ferner mindestens zwei Klemmflügel (50) aufweist, die senkrecht zur Längsrichtung des Grundkörpers (40) relativ zu dem Grundkörper (40) verschiebbar sind, wobei jeder Klemmflügel (50) einen Anschlag (51) aufweist, der sich in der Längsrichtung des Grundkörpers zumindest auf einer Seite des Grundkörpers über den Grundkörper hinaus erstreckt.

3. Gelenkinterimsprothese nach Anspruch 2, bei welcher jeder der Klemmflügel (50) über mindestens eine Teleskopstange (52), bevorzugt zwei Teleskopstangen, verschiebbar ist.

4. Gelenkinterimsprothese (1) nach Anspruch 3, bei der mindestens eine Teleskopstange (52) mindestens eines Klemmflügels (50) eine Ausrutschsicherung (53) aufweist, die bevorzugt als Aussparung in der Längsrichtung der Teleskopstange (52) ausgeführt ist, in die ein Klemmflügelfixationselement (56) des Grundkörpers (50) eingreift.

5. Gelenkinterimsprothese (1) nach einem der vorstehenden Ansprüche, bei welcher der Schaft in dem Durchgangsloch über ein Schaftfixationselement (43) fixierbar ist.

6. Gelenkinterimsprothese (1) nach einem der vorstehenden Ansprüche, bei welcher der Schaft (60) zumindest entlang eines Abschnitts seiner Länge einen Eingriffsabschnitt (62) für ein Kopplungswerkzeug (2) aufweist, der durch mindestens zwei parallele sich gegenüberliegende Flächen (65) ausgebildet ist.

7. Gelenkinterimsprothese (1) nach einem der vorstehenden Ansprüche, **dadurch gekennzeichnet, dass** sie einen zweiten Grundkörper (20) aufweist, wobei der zweite Grundkörper einen Gelenkankopplungsabschnitt (28) aufweist, an den ein Gelenkteil (10) mit seinem Gelenkkopplungsabschnitt (14) abnehmbar fixierbar ist.

8. Gelenkinterimsprothese (1) nach einem der vorstehenden Ansprüche, **dadurch gekennzeichnet, dass** sie ferner einen Auflageschaft (70) mit einem distalen und einem proximalen Ende aufweist, wobei zwischen dem distalen und dem proximalen Ende des Auflageschafts ein Auflageflansch (78) angeordnet ist, der sich ausgehend vom Auflageschaft radial nach außen erstreckt.

9. Gelenkinterimsprothese (1) nach Anspruch 8, bei welcher der Schaft (60) und der Auflageschaft (70) an ihrem proximalen Ende jeweils einen Kopplungsabschnitt (64, 74) und an ihrem gegenüberliegenden distalen Ende jeweils einen dazu komplementäre Kopplungsabschnitt (64, 74) und mindestens einen Eingriffsabschnitt für ein Kopplungswerkzeug aufweisen, wobei die Kopplungsabschnitte (64, 74) bevorzugt als Gewinde ausgeführt sind.

10. Gelenkinterimsprothese (1) nach einem der vorstehenden Ansprüche, umfassend eine antiadhäsive, antimikrobielle und/oder antibiotika-haltige Oberfläche oder Oberflächenanteile.

## Claims

1. Interim joint prosthesis (1) insertable into a long bone in order to replace a joint endoprosthesis that is to be explanted, wherein the interim joint prosthesis has a main body (40) with a main-body coupling segment (44) and a shaft (60) with a shaft coupling segment (64), and the shaft coupling segment (64) can be releasably coupled to the main-body coupling segment (44), **characterized in that** the main-body coupling segment (44) has a through-hole to permit the coupling, in which through-hole the shaft is arranged displaceably, such that the shaft can protrude on both sides of the main body (40) .

2. Interim joint prosthesis (1) according to Claim 1, in which the main body (40) moreover has at least two clamping wings (50) which are displaceable relative to the main body (40), perpendicularly with respect to the longitudinal direction of the main body (40), wherein each clamping wing (50) has a stop (51) which extends beyond the main body, in the longitudinal direction of the main body, at least on one side of the main body.

3. Interim joint prosthesis according to Claim 2, in which each of the clamping wings (50) is displaceable via at least one telescopic rod (52), preferably two telescopic rods.

4. Interim joint prosthesis (1) according to Claim 3, in which at least one telescopic rod (52) of at least one clamping wing (50) has an anti-slip device (53), which is preferably designed as a recess in the longitudinal direction of the telescopic rod (52), into which recess a clamping wing fixation element (56) of the main body (50) engages.

5. Interim joint prosthesis (1) according to one of the preceding claims, in which the shaft is fixable in the through-hole via a shaft fixation element (43).

6. Interim joint prosthesis (1) according to one of the preceding claims, in which the shaft (60) has, at least along a section of its length, an engagement segment (62) for a coupling tool (2), which engagement segment (62) is formed by at least two parallel surfaces (65) lying opposite each other.

7. Interim joint prosthesis (1) according to one of the preceding claims, **characterized in that** it has a second main body (20), wherein the second main body has a joint-coupling segment (28), to which a joint part (10) is releasably fixable with its joint-coupling segment (14).

8. Interim joint prosthesis (1) according to one of the preceding claims, **characterized in that** it moreover has a support shaft (70) with a distal end and a proximal end, wherein a support flange (78) is arranged between the distal end and the proximal end of the support shaft and extends radially outwards from the support shaft.

9. Interim joint prosthesis (1) according to Claim 8, in which the shaft (60) and the support shaft (70) each have, at their proximal end, a coupling segment (64, 74), and, at their opposite distal end, they each have a coupling segment (64, 74) complementary thereto and at least one engagement segment for a coupling tool, wherein the coupling segments (64, 74) are preferably configured as threads.

10. Interim joint prosthesis (1) according to one of the preceding claims, comprising an antiadhesive, antimicrobial and/or antibiotic-containing surface or surface portions.

## Revendications

1. Prothèse articulaire provisoire (1) pouvant être introduite dans un os tubulaire pour remplacer une endoprothèse articulaire à explanter, la prothèse articulaire provisoire présentant un corps de base (40) avec une portion d'accouplement de corps de base (44) et un arbre (60) avec une portion d'accouplement d'arbre (64) et la portion d'accouplement d'arbre (64) pouvant être accouplée de manière détachable à la portion d'accouplement de corps de base (44), **caractérisée en ce que** la portion d'accouplement de corps de base (44) présente pour l'accouplement un trou traversant dans lequel l'arbre est disposé de manière déplaçable de telle sorte que l'arbre puisse dépasser des deux côtés du corps de base (40).

2. Prothèse articulaire provisoire (1) selon la revendication 1, dans laquelle le corps de base (40) présente en outre au moins deux ailettes de serrage (50) qui peuvent être déplacées perpendiculairement à la direction longitudinale du corps de base (40) par rapport au corps de base (40), chaque ailette de serrage (50) présentant une butée (51) qui s'étend dans la direction longitudinale du corps de base, au moins d'un côté du corps de base jusqu'au-delà du corps de base.

3. Prothèse articulaire provisoire selon la revendication 2, dans laquelle chacune des ailettes de serrage (50) peut être déplacée par le biais d'au moins une tige télescopique (52), de préférence par le biais de deux tiges télescopiques.

4. Prothèse articulaire provisoire (1) selon la revendication 3, dans laquelle au moins une tige télescopique (52) d'au moins une ailette de serrage (50) présente une fixation anti-glissement (53) qui est réalisée de préférence sous forme d'évidement dans la direction longitudinale de la tige télescopique (52), dans laquelle s'engage un élément de fixation d'ailettes de serrage (56) du corps de base (50).

5. Prothèse articulaire provisoire (1) selon l'une quelconque des revendications précédentes, dans laquelle l'arbre peut être fixé dans le trou traversant par le biais d'un élément de fixation d'arbre (43).

6. Prothèse articulaire provisoire (1) selon l'une quelconque des revendications précédentes, dans laquelle l'arbre (60) présente, au moins le long d'une portion de sa longueur, une portion d'engagement (62) pour un outil d'accouplement (2), qui est réalisée par au moins deux surfaces parallèles opposées (65).

7. Prothèse articulaire provisoire (1) selon l'une quelconque des revendications précédentes, **caractérisée en ce qu'**elle présente un deuxième corps de base (20), le deuxième corps de base présentant une portion de raccordement d'articulation (28) au niveau de laquelle une partie d'articulation (10) peut être fixée de manière amovible avec sa portion d'accouplement d'articulation (14).

8. Prothèse articulaire provisoire (1) selon l'une quelconque des revendications précédentes, **caractérisée en ce qu'**elle présente en outre un arbre de support (70) avec une extrémité distale et une extrémité proximale, une bride de support (78) étant disposée entre l'extrémité distale et l'extrémité proximale de l'arbre de support, laquelle s'étend radialement vers l'extérieur à partir de l'arbre de support.

9. Prothèse articulaire provisoire (1) selon la revendication 8, dans laquelle l'arbre (60) et l'arbre de support (70) présentent au niveau de leur extrémité proximale à chaque fois une portion d'accouplement (64, 74), et au niveau de leur extrémité distale opposée, à chaque fois une portion d'accouplement (64, 74) complémentaire à celle-ci, et au moins une portion d'engagement pour un outil d'accouplement, les portions d'accouplement (64, 74) étant de préférence réalisées sous forme de filetage.

10. Prothèse articulaire provisoire (1) selon l'une quelconque des revendications précédentes, comprenant une surface ou des parties de surface antiadhésives, antimicrobiennes et/ou contenant des antibiotiques.
